(19) Europäisches Patentamt
European Patent Office
Office européen des brevets

(11) Publication number: 0 158 319
A2

# (12) EUROPEAN PATENT APPLICATION

(21) Application number: 85104277.0

(22) Date of filing: 09.04.85

(51) Int. Cl.[4]: C 07 D 471/08
C 07 D 487/08
//(C07D471/08, 221:00, 221:00),
(C07D487/08, 241:00, 241:00)

(30) Priority: 10.04.84 US 598585

(43) Date of publication of application:
16.10.85 Bulletin 85/42

(84) Designated Contracting States:
AT BE CH DE FR GB IT LI LU NL SE

(71) Applicant: UNION CARBIDE CORPORATION
Old Ridgebury Road
Danbury Connecticut 06817(US)

(72) Inventor: Budnik, Richard Anthony
77 Carpenter Avenue, Apt. 3M
Mt. Kisco New York 10549(US)

(72) Inventor: Sandner, Michael Ray
77 West Orchard Road
Chappaqua New York 10514(US)

(74) Representative: Wuesthoff, Franz, Dr.-Ing. et al,
Patentanwälte Wuesthoff -v. Pechmann-Behrens-Goetz
Schweigerstrasse 2
D-8000 München 90(DE)

(54) **Synthesis of 1-Azabicyclo(2.2.2) octanes or 1,4-Diazabicyclo(2-2.2)octanes.**

(57) A method of preparing 1-azabicyclo[2.2.2]octanes or 1,4-diazabicyclo[2.2.2.]octanes by contacting acyclic or heterocyclic amines with a high-silica zeolite having a silica to alumina ratio of at least 20 to 1.

EP 0 158 319 A2

# SYNTHESIS OF 1-AZABICYCLO[2.2.2] OCTANES OR 1,4-DIAZABICYCLO[2.2.2]OCTANES

## FIELD OF THE INVENTION

This invention relates to the preparation of bicyclic amines compounds. More particularly, this invention relates to an improved method for the production of 1-aza and 1,4-diaza bicyclo[2.2.2]octanes and is particularly well suited for the production of 1-aza and 1,4-diazabicyclo[2.2.2]octane and C-substituted 1-aza and 1-4,diazabicyclo[2.2.2]octane by using high-silica zeolite as catalysts to give increased selectivity to the bicyclo-amine products and a reduction in the number of undesired by-products.

## DESCRIPTION OF THE PRIOR ART

1,4-diazabicyclo[2.2.2]octane has been prepared by cyclizing an amine such as ethylenediamine, diethylenetriamine, triethylenetetramine, N-hydroxyethylpiperazine or N-aminoethylpiperazine in the presence of a silica, alumina or tungsten catalyst. However, such reactions have not been entirely satisfactory for a number of reasons. For example, the reaction conditions are so severe that said catalysts promote cracking and condensation reactions of the reactants and the reaction products. This results in the product being obtained in small yields. In addition, the reaction product is characterized by the presence of a wide variety of undesired products. This results in poor yields of 1,4-diazabicyclo[2.2.2]octane and poor process and

economics due to the expense and care that is necessary to obtain relatively pure 1,4-diazabicyclo[2.2.2]octane from the reaction mixture containing by-products. Among the undesirable by-products formed utilizing the aforesaid catalyst are piperazines, N-alkylpiperazines and a variety of pyrazine derivatives. These by-products have physical and chemical properties closely related to those 1,4-diazabicyclo[2.2.2]octane (hereinafter "DABCO"). Therefore, physical separation of the by-products from DABACO by conventional techniques such as distillation or fractional crystallization, are only partially effective.

Low-silica zeolite,i.e those having a silica to alumina ratio less than 10 have been described as catalysts for making DABCO from amines. For example, Japanese patent No. 5-0058-096 described the use of molecular sieves having a low silica to alumina ratio, as catalysts to form DABCO from (β-aminoethyl)-piperazine and hydrogen. However, it is reported that such a process produces low selectivity to DABCO. Also, U.S. Patent 3,903,079 discloses a process for preparing heterocyclic amines from acyclic compounds using low-silica zeolites such a zeolites X or Y. Additionally, U.S. patent 3,728,408 describes the reaction of ethylene oxide with ammonia in the presence of an aluminosilicate zeolite having a composition wherein the $SiO_2/Al_2O_3$ ratio is at least 5 to produce products, such as morpholine, piperazine and cyclic ethers (dioxane).

In addition, a variety of non-zeolite catalysts have been described for preparing cyclic amines from piperazine. For example, U.S. Patent 2,937,176 describes the use of kaolin while U.S. Patents 3,297,701, 3,342,820 and 4,038,038 describe the use of phosphate catalysts. In addition, kaolin has been used to catalyze the reaction between piperazine and ethylene oxide (U.S.Patent 3,772,293). Further alumina catalysts for the preparation of bicyclic amines have also been described in Japanese patent 50117797, Belgium patent 846,634 and German patent 2442929.

The present invention provides a new process for the production of 1-aza or 1,4-diazabicyclo[2.2.2]octane and particularly the production of DABCO or derivatives thereof by contacting at least one acyclic or heterocyclic amines or mixtures thereof with a high-silica zeolite resulting in improved selectivities to 1-aza or 1,4-diazabicyclo[2.2.2]octane products as well as reduced undesired by-products, in contrast to the prior art processes previously described.

SUMMARY OF INVENTION

The invention relates to a novel process for the preparation of 1-azabicyclo[2.2.2]octane or 1,4-dazabicyclo[2.2.2]octanes from acyclic or heterocyclic amines or mixtures thereof by contacting said feedstock with a high-silica zeolite catalyst at a temperature between about 250°C and 550°C.

DETAILED DESCRIPTION OF THE INVENTION

The process of the present invention provides for the conversion of acyclic or heterocyclic amines or mixtures thereof to 1-azabicyclo[2.2.2.]octanes or 1,4-diazabicyclo[2.2.2.]octanes and results in improved selectivity to the 1-azabicyclo[2.2.2.]octanes or 1,4-diazabicyclo[2,2,2,]octanes products and a reduction in the formation undesirable by-products, such as ethylenediamine, ethanolamine, morpholine or dioxane. The present process provides selectivity to the 1-azabicyclo[2.2.2]octanes or 1,4-diazabicyclo[2.2.2.]octanes products of at least 60 percent by weight, preferably at least 70 percent by weight.

In particular, the present invention provides a process for the production of 1-azabicyclo[2.2.2.]octanes having the formulae:

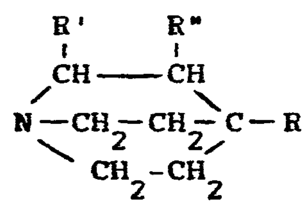

wherein: R' is selected from the group consisting of alkyl groups containing 1 to 4 carbon atoms or alkenyl groups containing 1 to 4 carbon atoms or preferably hydrogen;
R" is selected from the group consisting of alkyl groups containing 1 to 4 carbon atoms or alkenyl groups containing 1 to 4 carbon atoms or preferably hydrogen;
R is selected from a group consisting of alkyl

groups containing 1 to 12 carbon atoms or alkenyl groups containing 1 to 12 carbon atoms or preferably hydrogen or $-CH_2CH_2NH_2$ or $-CH_2CH_2OH$.

In particular, the present invention provides a process for the production of the above 1-azabicyclo[2.2.2.]octanes by contacting a high-silica zeolite with a heterocyclic amine or mixtures thereof. Representative of such amines are those amines having the formulae:

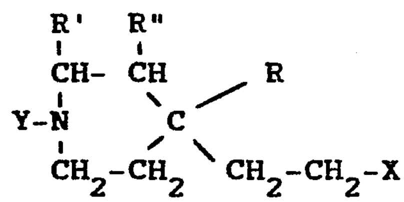

wherein: R' is defined as above;

R" is defined aa above;

R is defined as above;

X is preferably $NH_2$ or more preferably OH or $-CH_2CH_2OH$ or $-CH_2CH_2NH_2$;

Y is preferably hydrogen or $-CH_2CH_2OH$ or $-CH_2CH_2NH_2$.

In particular the present invention also provides a process for the production of 1,4-diazabicyclo[2.2.2.]octanes having the formulae:

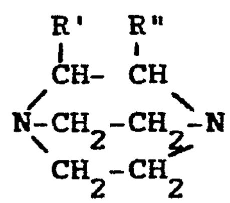

wherein: R' is defined as above;

R" is defined as above.

In particular the present invention provides a process for the production of the above 1,4-diazabicyclo[2.2.2.]octanes by contacting a

high-silica zeolite with an acyclic or heterocyclic amine or mixtures thereof. Representative of such amines are those amines having the formulae:

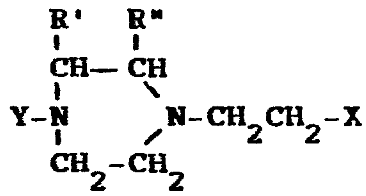

wherein: R' is defined as above;
R" is defined as above;
Y is defined as above;
X is defined as above;

or

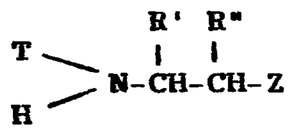

wherein: R' is defined as above;
R" is defined as above;
Z is $NH_2$ or OH;
T is selected from a group consisting of hydrogen or $-CH_2CH_2W$; and W is -OH or $-NH_2$ or $-NHCH_2CH_2OH$ or $-NHCH_2CH_2NH_2$.

Representative of amines suitable for production of 1-azabicyclo [2.2.2.]octanes according to the present invention include 4-(β-hydroxyethyl)piperidine and N-(β-aminoethyl)piperidine. Representative of amines suitable for production of 1,4-diazabicyclo [2.2.2.]octanes according to he present invention include N-(β-hydroxyethyl)piperazine, N-(β-aminoethyl)piperazine and N-(β-aminoethyl)ethanolamine.

The catalysts employed in the invention utilize the properties of high-silica zeolites, i.e.

those having a $SiO_2/Al_2O_3$ mole ratio which exceeds about 20. Particularly effacious zeolites are those having silicia to alumina ratios in the range of about 35 to about 85 and preferably in the range of about 35 to 55. In general, any zeolite (a crystalline material having a intra crystalline void volume) having a silica to alumina ration greater than about 20 are suitable as the high-silica zeolites employed in he process of this invention.

Representative of these high-silica zeolites are those commercially available materials such as but not limited thereto are "Silicalite", ZSM-5, ZSM-8, ZSM-11, ZSM-12, Hyper Y, ultrastabilized Y, hereinafter designated "ultra-Y", and the like.

"Silicalite" (A Trademark of Union Carbide Corporation) is a crystalline silica composition having a hydrophobic/organophilic characteristic which permits its use for selectively adsorbing organic materials preferentially to water. Silicalite is more completely described in U.S. Patent 4,061,724, assigned to Union Carbide Corporation. It is described in said patent as silica polymorph consisting of crystalline silica, said silicia polymorph after calcination in air at 600°C for 1 hour, having a mean refractive index of 1.39 ± 0.01 and a specific gravity at 25°C of 1.70 ± 0.05 g/cc and as a silica polymorph after calcination in air at 600°C for 1 hour having as the six strongest d-values of its X-ray powder diffraction pattern those set forth in Table A.

Table A

| d(A) | Relative Intensity[a] |
|---|---|
| 11.1 ± 0.2 | VS |
| 10.1 ± 0.2. | VS |
| 3.85 ± 0.07 | VS |
| 3.82 ± 0.07 | S |
| 3.76 ± 0.05 | S |
| 3.72 ± 0.05 | S |

(a) VS = Very Strong; S = Strong

The preparation of silicalite is set forth in Examples 3, 5, 6 and 7 of U.S. Patent 4,061,724, which examples are incorporated herein by reference.

The above-mentioned ZSM-type zeolites are described in various U.S. Patents and Foreign Patents as follows:

ZSM-5 is a crystalline zeolite and is disclosed in U.S. Patent 3,702,886. The preparation of ZSM-5 is set forth in Examples 1, 2, 6, 22, 26 and 27 of U.S. Patent 3,702,886, which examples are incorporated herein by reference.

ZSM-8 is a crystalline zeolite and is disclosed in British Specification 1,334,243, published October 17, 1973.

ZSM-11 is a crystalline zeolite and is disclosed in U.S. Patent 3,709,979. Its preparation is set forth in Examples 1, 2, 4, 5, 8 and 10 of this patent, which examples are incorporated herein by reference.

ZSM-12 is a crystalline zeolite and is disclosed in U.S. Patent 3,832,449. The preparation of ZSM-12 is set forth in Examples I, II, III, IV,

V, VI, VII and VIII of this patent which examples are incorporated herein by reference.

Ultrastabilized Y is a form of zeolite Y which has a silica to alumina ratio suitable for use in the present invention. A description of ultrastabilized Y is found in "Crystal Structures of Ultra-stable Faujasites", Advances in Chemistry Series, No. 101, American Chemical Society, Washington, DC, pages 226-278 (1971).

In addition, a class of high-silica zeolites designated and hereinafter referred to as Catalyst (1) is the zeolites used in the present examples.

Catalyst (1) comprises a class of zeolites, described more fully in co-pending U.S. Patent application Serial No. 655,065, filed February 4, 1976. This class of zeolites comprises zeolite compositions which are prepared in an organic-free reaction mixture. These zeolite compositions are highly siliceous and can contain an extraordinarily high amount of monovalent or divalent metal cations. Further, the hydrophobicity of these zeolite compositions make them ideally suited for use in the present invention. The Catalyst (1) compositions exhibit an aluminosilicate crystal structure wherein at least some of the $AlO_4^{-2}$ tetrahedra thereof are associated with, and electrovalently neutralized by a metal cation. The composition of these zeolites in the dehydrated state can be expressed empirically in terms of the moles of oxides as follows:

$$0.01\text{-}2.0\ M_{\frac{2}{n}}O \quad : \quad Al_2O_3 \quad : \quad 20\text{-}100\ SiO_2$$

wherein M represents at least one metallic cation and n represents the valence of M as prepared from reaction mixtures free of organic cations, as hereinafter described.

These zeolites may also be exchanged with other cations, including metal ions, hydrogen ions, rare earth metal ions, and mixtures thereof, by contacting the zeolite with solutions containing or or more of the desired cations.

In conjunction with the aforesaid chemical composition, these zeolites, i.e. Catalyst (1) compositions, possess a distinguishing crystalline structure characterized by an X-ray powder diffraction pattern having at least the following interplanar spacings:

Table B

| Interplanar Spacing, d (A) |
|---|
| 11.1 ± 0.2 |
| 10.1 ± 0.2 |
| 3.85 ± 0.07 |
| 3.74 ± 0.05 |
| 3.72 ± 0.05 |

These values were determined by standard techniques as follows. The radiation was the K-alpha doublet of copper, and a scintillation-counter spectrometer with a strip-chart pen recorder was used. The peak heights and the peak or line position as a function of two times theta (Θ), where theta is the Bragg angle, were read from the spectrometer chart. From these, the relative intensities and d (observed),

the interplanar spacing in A, corresponding to the recorded lines, were determined.

Ion-exchange of the original cations by other cation species does not substantially alter the X-ray pattern of Catalyst (1), but some minor shifts in interplanar spacing and variations in relative intensity can occur. Other minor variations can occur depending on the silica-to-alumina ratio of the particular sample and whether or not the sample had been subjected to elevated temperatures. In any event the d-spacings of the X-ray pattern will be within the tolerances indicated in Table B.

In conjunction with the aforesaid chemical composition and X-ray powder diffraction pattern, Catalyst (1) compositions exhibit certain distinguishing infrared absorption characteristics. Infrared analytical techniques are recognized as highly useful in the study of crystalline zeolites: see for example U.S. Patents 3,506,400 and 3,591,488 to Eberly et al., issued April 14, 1970 and July 6, 1971, respectively, and E. H. Flanigen, H. Khatami and H. A. Szymanski, "Advanced Chemical Series"., Vol. 101, 1971 (page 201 et seq.).

The infrared analysis spectra were obtained on a Perkin-Elmer Model 112 single-beam instrument for the hydroxyl-stretching region 3800-3000 $cm^{-1}$, on a Perkin-Elmer Model 621 double-beam instrument for both the mid-infrared region 1600-1300 $cm^{-1}$ and the framework region 1300-3000 $cm^{-1}$. After calcination at 600°C in air, the samples were run as self-supported wafers (20 mg.), and the spectra in

the hydroxyl-stretching region were obtained after thermal treatments at 200°C in vacuum for two hours.

Specific high-silica zeolite catalyst compositions which are preferred in the process of the present invention include catalysts comprising Catalyst (1) with at least one ion such as of Na, K, Mg, Zn, Ca, $UO_2$ or Cu introduced therein. These preferred catalysts are hereinafter designated by the abbreviations such as Na-Cat(1), K-Cat(1) or $UO_2$-Cat(1), wherein these abbreviations designate metal ions introduced directly into the framework of Catalyst (1). In addition, the ZSM-5 type zeolites, i.e., ZSM-5, ZSM-8, ZSM-11 and ZSM-12, hereinbefore described, are preferred catalysts for the process of the present invention and may also contain metals or metal ions.

The reaction conditions of temperature and pressure to be employed are within the range of from about 250°C to about 550°C and from about 0.5 to about 2 atmospheres absolute and preferably within the range of from about 350°C to about 450°C and from about 0.8 to about 1.2 atmospheres absolute. It is generally preferable to carry out the reaction in the vapor phase with a space velocity (SV) in the range of about 200 $h^{-1}$ to about 2500 $h^{-1}$, preferably from about 200 $hr^{-1}$ to about 1000 $h^{-1}$ and most preferably from about 200 $h^{-1}$ to about 600 $h^{-1}$. It will be understood that yields tend to increase as the space velocity decreases. Contact times between about 0.2 to about 3 seconds may be employed.

If desired, the amine reactant(s) may be dissolved in a suitable solvent and sent to the reactor as a liquid feed. The LHSV (liquid hourly space velocity in terms of volume of liquid per volume of catalyst per hour) of the liquid feed should be from about 1 to about 8, preferably from about 1 to about 4. The solvent which may be used herein should be inert and should not be reactive with the reactant(s) or product(s). Among the solvents to be specially considered are water and hydrocarbons, such as light aromatics, aliphatic and naphthenic minerals oils, and alkylated benzene or alkylated naphthlene.

The starting amines employed herein may be substantially anhydrous. However, aqueous amines may be used due to the hydrophobic character of the high-silica zeolites employed in the instant process.

EXPERIMENTAL PROCEDURE

The process of the invention was carried out using a standard single pass, trickle bed reactor comprising a pyrex glass tube (15 mm internal diameter) with a (three inch) 7,62 cm long precatalyst zone therein packed with glass beads followed by the catalyst bed (approximately 2 grams) held in place by quartz sand. The remainder of the reactor comprises a (two inch) 5,08 cm post-catalyst zone packed with glass beads.

The reactor is wrapped with heating wire and its temperature is controlled by use of a thermocouple inserted in the middle of the catalyst bed.

In carrying out the process, the liquid reactants were fed dropwise from a metered addition funnel placed above the reactor. The reactant was passed through the reactor using argon as a carrier gas at a flow rate of about 44 $cm^3$/min.

The packed trickle bed reactor was preheated to about 400°C under a continuous argon purge (about 44 $cm^3$/min) for about thirty minutes. It was observed that at temperatures below about the boiling point of the reactant, the reactor operated as a trickle bed reactor while at temperatures above the boiling point of the reactant the reactant is vaporized in the precatalyst section of the reactor and is swept through the catalyst bed by the argon carrier gas.

The overall conversion to products, i.e. bicyclic amines, increases with increasing reactor temperature but selectivity decreases.

In accordance with the process of the present invention a high-silica zeolite, hereinbefore described as Catalyst (1) is prepared such that the silica to alumina ratio is about 20 to 1 or greater. This high-silica zeolite is prepared by initially forming a reaction mixture, as follows, by:

(a) dissolving 899 g (1.98 pounds) of reagent-grade NaOH in 5.99 kg (13.2 pounds) of water at 95°C with agitation;

(b) dissolving 540 g (1.19 pounds) of sodium aluminate into the solution of (a);

(c) thoroughly mixing 24.7 kg (54.5 pounds) of

an aqueous colloidal silica sol, and (120 pounds) [54,4 kg] of water in a (100-gallon) [379,1 l] kettle;

(d) mixing (1.98 pounds) [899 g] of a "ZSM-5 type" zeolite crystals with (2 pounds) [907 g] of water;

(e) mixing hot solution of (b) into the silica-water mixture of (c);

(f) adding the crystal-water mixture of (d) to the mixture of (e); and

(g) agitating the mixture of (f) for about 5 minutes.

The reaction mixture so formed above is then maintained at a temperature of about 150°C for about 120 hours. The solid reaction product formed is separated from the liquor by filtration, then washed with (100 gallons) [379,1 l] of water and dried. Chemical analysis of the reaction product formed from this above reaction mixtures has the following composition:

| | |
|---|---|
| Wt. percent $Na_2O$ | 3.1 |
| Wt. percent $Al_2O_3$ | 3.3 |
| Wt. percent $SiO_2$ | 82.0 |
| Wt. percent C | 0.8 |
| Wt. percent N | 0.06 |
| Loss on Ignition (LOI) | 9.2 |

X-ray powder diffraction analysis of the reaction product was identified as a zeolite having a characteristic X-ray powder diffraction pattern containing at least the d-spacings of Table B.

A high-silica zeolite was prepared according to the above method for preparing Catalyst (1) (hereinafter abbreviated Cat (1)). Catalyst (1) was then acid or metal exchanged to give the

catalysts having the silica to alumina ratio of Catalyst (1) as follows: Na-Cat(1), H-Cat(1), K-Cat(1), Mg-Cat(1), Zn-Cat(1), Ca-Cat(1), $UO_2$-Cat(1) and Cu-Cat(1).

These catalysts were then washed repeatedly with hot water to remove any excess salts and then dried and calcined before use.

EXAMPLES

Bicyclic amines, such as 1,4-diayabicyclo[2.2.2.]octane and 1-azabicyclo[2.2.2.]octane (hereinafter referred to as quinuclidine) were prepared by the process of this invention. To prepare 1,4-diazabicyclo[2.2.2.]octane the reactants were N-(β-hydroxyethyl)-piperazine or N-(β-aminoethyl)-piperazine, or N-(β-aminoethyl)ethanolamine. The preferred reactant was N-(β-hydroxyethyl)-piperazine.

The following Examples illustrate the process of the invention and are given by way of illustration and not as a limitation on the scope of the invention.

Examples 1-6:

Catalyst H-Cat(1) and K-Cat(1), alumina, and kaolin were used to prepare DABCO from N-(β-hydroxyethyl)-piperazine according to the aforementioned experimental procedure. The catalyst activity and selectivity to 1,4-diazabicyclo[2.2.2.]octanes are dependent, in part upon reactor design and selected operating conditions so that examples 1 to 6 were carried out

in essentially the same manner by employing a temperature of about 400°C and a space velocity of about 1900 ± 100 $h^{-1}$.

The date of TABLE I shows the improvement in selectivity for the formation of DABCO using the high silica zeolite catalyst of this invention as compared to the H- or K- alumina or a kaolin catalyst.

Examples 1-6 show that the selectivity to DABCO decreased in the order: H-Cat(1) : Silicate : Kaolin : H-alumina. The same relative selectivity is observed using the catalysts in their potassium form.

TABLE I

Mole Percents [a,b]

| Example | Catalyst | DABCO | Piperazine | Other | Overall % Conversion |
|---|---|---|---|---|---|
| 1 | H-Cat(1) | 87 | 13 | 0 | 9.7 |
| 2 | Silicalite | 76 | 18 | 6 | 4.8 |
| 3 | Kaolin | 66 | 25 | 9 | 14.3 |
| 4 | H-Alumina | 47 | 34 | 20 | 95.6 |
| 6 | K-Cat(1) | 89 | 11 | 0 | 8.7 |
| 6 | K-Alumina | 39 | 47 | 14 | 97.5 |

(a) Conversion at constant space velocity (1900 ± 100 $h^{-1}$)

(b) The feedstock was N-(β-hydroxyethyl)-piperazine (HEP) and the reactor was heated to about 400°C.

Example 7-20

These Examples show the effect of high conversions on reaction selectivity.

TABLE II shows that selectivity to DABCO is essentially insensitive to changes in overall conversion in the range of about 7 to about 96% for H-alumina (Examples 7 and 8) and from about 10-21% for H-Cat(1) (Examples 10 and 11). Ca-Cat(1) shows only a slight change in selectivity to DABCO as the overall conversion increased from about 9% to about 72% (Examples 11-15 and 18-20).

Examples 21-38

These Examples show the effect on selectivity to product for different cation exchanged catalysts.

The data of TABLE III show the relative selectivity of Catalyst(1) when cation exchanged with different cations. Examples 31, 32 and 38 show acid exchanged alumina. Examples 21 and 33 show the relative selectivity when no catalyst is employed. The zeolites prepared from monovalent cations, i.e. $K^+$, $H^+$, and $Na^+$, were more selective in producing DABCO than those prepared from divalent cations, e.g. $Mg^{2+}$, $Zn^{2+}$, $Ca^{2+}$, $UO_2^{2+}$ and $Cu^{2+}$.

Examples 39-42

These Examples describe various reactants using Ca-Cat (1) to prepare DABCO and piperazine or quinuclidine.

The data of Table IV show that the formation of quinuclidine from

N-(β-hydroxyethyl)-piperidine was more selective and gave higher conversions than the formation of DABCO from N-(β-hydroxyethyl)-piperazine.

TABLE II

| Example | Catalyst | Mole Percent Products[f] DABCO | Piper-azine | Others | % Conversion | Catalyst[a] Wt. | Argon[b] Flow | HEP Feed Rate[c] | SV[d,g] |
|---|---|---|---|---|---|---|---|---|---|
| 7 | H-Alumina | 47 | 33 | 20 | 95.6 | 2.0 | 2400 | 18.2 | 1942 |
| 8 | H-Alumina | 43 | 27 | 30 | 6.6 | 0.1 | 2640 | 18.2 | 40434 |
| 9 | H-Cat (1) | 87 | 10 | 3 | 21.0 | 8.0 | 4800 | 18.2 | 685 |
| 10 | H-Cat (1) | 87 | 13 | 0 | 9.7 | 2.0 | 2580 | 16.0 | 1864 |
| 11 | Ca-Cat (1) | 70 | 16 | 14 | 72.4 | 10.0 | 1800 | 8.7 | 230 |
| 12 | Ca-Cat (1)[e] | 72 | 16 | 12 | 69.9 | 10.0 | 1800 | 8.7 | 230 |
| 13 | Ca-Cat (1) | 70 | 17 | 13 | 59.2 | 8.0 | 2700 | 18.2 | 510 |
| 14 | Ca-Cat (1) | 71 | 19 | 10 | 40.3 | 8.0 | 10800 | 18.2 | 1185 |
| 15 | Ca-Cat (1) | 75 | 17 | 7 | 21.1 | 8.0 | 43200 | 18.2 | 3885 |
| 16 | Ca-Cat (1) | 90 | 10 | 0 | 4.4 | 2.0 | 1800 | 7.3 | 1057 |
| 17 | Ca-Cat (1) | 83 | 14 | 3 | 7.3 | 2.0 | 1200 | 7.3 | 857 |
| 18 | Ca-Cat (1) | 71 | 12 | 17 | 8.6 | 2.0 | 600 | 7.3 | 657 |
| 19 | Ca-Cat (1) | 69 | 19 | 11 | 11.1 | 2.0 | 1800 | 7.3 | 1057 |
| 20 | Ca-Cat (1) | 70 | 13 | 17 | 11.6 | 2.0 | 300 | 7.3 | 557 |

(a) g
(b) $cm^3$/hr
(c) g/hr (feedstock was N-(β-hydroxyethyl)-piperazine, i.e., HEP)
(d) Space velocity (SV) in $h^{-1}$.
(e) Example 12 is an average of 12.5 hrs. of continuous operation.
(f) Examples 7-20 conducted at 400°C with contact times of about 1 second.
(g) Table II, supra, shows the relationship of space velocity to percent conversion. Overall conversions of greater than 50% may be achieved by employing a space velocity of from about 200 $h^{-1}$ to 500 $hr^{-1}$.

TABLE III[a]

| Example | Catalyst | Starting[b,e] Material | Mole Percent Products[d] DABCO | Piperazine | Others[c] | Overall % Conversion | Conversion To DABCO, % |
|---|---|---|---|---|---|---|---|
| 21 | None | OH | 0 | 0 | 0 | 0 | 0 |
| 22 | Ca-Cat (1) | OH | 90 | 10 | 0 | 4.1 | 3.7 |
| 23 | K-Cat (1) | OH | 89 | 11 | 0 | 8.7 | 7.7 |
| 24 | H-Cat (1) | OH | 87 | 13 | 0 | 9.7 | 8.4 |
| 25 | Na-Cat (1) | OH | 81 | 13 | 6 | 9.6 | 7.8 |
| 26 | Mg-Cat (1) | OH | 76 | 19 | 5 | 6.4 | 4.9 |
| 27 | Zn-Cat (1) | OH | 71 | 22 | 7 | 7.3 | 5.2 |
| 28 | Ca-Cat (1) | OH | 69 | 19 | 11 | 11.1 | 7.7 |
| 29 | $UO_2$-Cat (1) | OH | 66 | 23 | 11 | 8.4 | 5.5 |
| 30 | Cu-Cat (1) | OH | 63 | 24 | 13 | 9.4 | 5.9 |
| 31 | H-Alumina | OH | 47 | 34 | 20 | 95.6 | 44.9 |
| 32 | H-Alumina (diluted X20) | OH | 43 | 27 | 30 | 6.6 | 2.8 |
| 33 | None | $NH_2$ | 0 | 0 | 0 | 0 | 0 |
| 34 | Ca-Cat (1) | $NH_2$ | 47 | 53 | 0 | 1.7 | 0.8 |
| 35 | H-Cat (1) | $NH_2$ | 39 | 57 | 4 | 4.8 | 1.9 |
| 36 | Zn-Cat (1) | $NH_2$ | 38 | 53 | 9 | 7.2 | 2.7 |
| 37 | Cu-Cat (1) | $NH_2$ | 31 | 50 | 19 | 5.0 | 1.6 |
| 38 | H-Alumina | $NH_2$ | 14 | 67 | 19 | 28.9 | 4.0 |

(a) Temperature about 400°C

(b) OH = N-(β-hydroxyethyl)-piperazine, $NH_2$ = N-(β-aminoethyl)-piperazine

(c) Mixture containing in part ethylenediamine, ethanolamine, dioxane and morpholine

(d) The relative selectivity ordering for Ca-Cat(1), H-Cat(1), Zn-Cat(1) and Cu-Cat(1) did not change as the feedstock was changed from N-(β-hydroxyethyl)-piperazine to N-(β-aminoethyl)-piperazine.

(e) Space velocity of 2000 $h^{-1}$

TABLE IV

| Example | Reactant | % Reaction[a] | Products: DABCO | Products: Piperazine | Products: Other | Products: Quinuclidine | Feed Rate[b] | Contact Time ( s ) |
|---|---|---|---|---|---|---|---|---|
| 39 | N-(β-hydroxyethyl)-piperazine | 4.1 | 90 | 10 | 0 | 0 | 3.64 | 1.1 |
| 40 | N-(β-hydroxyethyl)-piperidine | 23 | 0 | 0 | 0 | 100 | 1.04 | 0.3 |
| 41 | N-(β-aminoethyl)-piperazine | 1.7 | 47 | 53 | 0 | 0 | 6.81 | 0.8 |
| 42 | N-(β-aminoethyl)-ethanolamine | 6.5 | 37 | 39 | 24[c] | 0 | 8.21 | 0.6 |

(a) % of reactant converted to products at 400°C (the reactor temperature).

(b) g reactant/g catalyst h

(c) A mixture of: ethylenediamine (18%); ethanolamine (5%); and morpholine (1%).

WHAT IS CLAIMED:

1. **A method for the production of 1-azabicyclo[2.2.2]octanes comprising contacting an amine having the formula:**

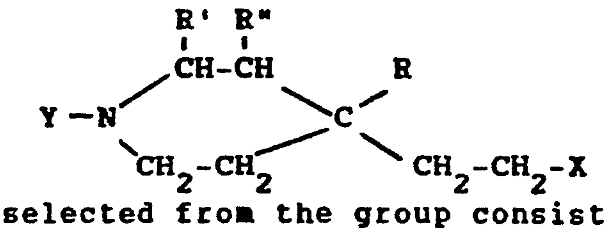

**wherein: R' is selected from the group consisting of hydrogen or alkyl groups containing 1 to 4 carbon atoms or alkenyl groups containing 1 to 4 carbon atoms; R" is selected from the group consisting of hydrogen or alkyl groups containing 1 to 4 carbon atoms or alkenyl groups containing 1 to 4 carbon atoms; R is selected from a group consisting of hydrogen or $-CH_2CH_2NH_2$ or $-CH_2CH_2OH$ or alkyl groups containing from 1 to 12 carbon atoms or alkenyl groups containing from 1 to 12 carbon atoms; Y is hydrogen or $-CH_2CH_2OH$ or $-CH_2CH_2NH_2$; X is $-OH$ or $-NH_2$ or $-CH_2CH_2OH$ or** $-CH_2CH_2NH_2$; with a high silica zeolite having a silica to alumina ratio of at least 20, preferably a ZSM-5 type zeolite, at a temperature of from 250°C to 550°C and at a space velocity of from 200 $h^{-1}$ to 2500 $h^{-1}$, preferably of from 200 $h^{-1}$ to 500 $h^{-1}$ and the selectivity to 1-azabicyclo[2.2.2]octanes being at least 70 mole percent of the product formed.

2. **The process of claim 1 wherein the temperature is from about 350°C to about 450°C.**

3. **The process of claim 2 wherein said zeolite has the following composition in the dehydrated state**

$$0.01\text{-}2.0\ M_{\frac{2}{n}}O \quad : \quad Al_2O_3 \quad : \quad 20\text{-}100\ SiO_2$$

wherein M represents at least one cation or a hydrogen cation and n represents the valence of M, said composition having a characteristic X-ray powder diffraction pattern having at least the interplanar spacings of Table B.

4. The process of claim 1 wherein the amine is selected from a group consisting of 4-(β-hydroxyethyl)piperidine or 4-(β-aminoethyl)piperidine or mixtures thereof.

5. The process of claim 3 wherein the zeolite has a silica to alumina ratio of about 35 to about 85, preferably of from 35 to 55.

6. The process of claim 1 wherein said 1-azabicyclo[2.2.2.]octanes are 1-azabicyclo[2.2.2.]octane.

7. The process of claim 3 wherein M is a monovalent or divalent metallic cation or $UO_2^{+2}$ or mixtures thereof, preferably a monovalent cation or selected from the group consisting of $Na^+$, $K^+$, $Ca^{+2}$, $Mg^{+2}$, $Zn^{+2}$, $Cu^{+2}$, $UO_2^{+2}$ or $H^+$.

8. A method for the production of 1,4-diazabicyclo[2.2.2.]octanes comprising contacting an amine having the formulae:

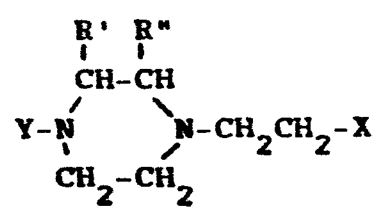

wherein: R' is defined as in claim 1;
R" is defined as in claim 1;
Y is defined as in claim 1;
X is defined as in claim 1;

or

```
            R' R"
            |  |
      T-NH-CH-CH-Z
```

wherein: R' is defined as in claim 1;
R" is defined as in claim 1;
Z is $-NH_2$ or -OH;
T is selected from a group consisting of hydrogen or $-CH_2CH_2W$; and W is -OH or $-NH_2$ or $-NHCH_2CH_2OH$ or $-NHCH_2CH_2NH_2$;

with a high silica zeolite having a silica to alumina ratio of at least 20,*a ZSM-5 type zeolite, at a temperature of from 250°C to 550°C at a space velocity of from 200 $h^{-1}$ to 2500 $h^{-1}$, preferably of from 200 $h^{-1}$ to 500 $h^{-1}$ the selectivity to 1,4-diazabicyclo/‾2.2.2_7octanes being at least 70 mole percent of the products formed.

*preferably

9. The process of claim 8 wherein the temperature is from about 350°C to about 450°C.

10. The process of claim 9 wherein said zeolite has the following composition in the dehydrated state

$$0.01\text{-}2.0\ M_{\frac{2}{n}}O \quad : \quad Al_2O_3 \quad : \quad 20\text{-}100\ SiO_2$$

wherein M represents at least one cation or a hydrogen cation and n represents the valence of M, said composition having a characteristic X-ray powder diffraction pattern having at least the interplanar spacings of Table B.

11. The process of claim 8 wherein the amine is selected from a group consisting of N-(β-hydroxyethyl)piperazine, or N-(β-aminoethyl)piperazine or N-(β-aminoethyl)ethanolamine or mixtures thereof.

12. The process of claim 10 wherein the zeolite has a silica to alumina ratio of 35 to 85, preferably of from 35 to 55.

13. The process of claim 8 wherein said 1,4-diazabicyclo[2.2.2.]octanes are 1,4-diazabicyclo[2.2.2.]octane.

14. The process of claim 10 wherein M is a monovalent or divalent metallic cation or $UO_2^{+2}$ or mixtures thereof, preferably a monovalent metallic cation or selected from the group consisting of $Na^+$, $K^+$, $Ca^{+2}$, $Mg^{+2}$, $Zn^{+2}$, $Cu^{+2}$, $UO_2^{+2}$ or $H^+$.

15. In the process for preparing 1-azabicyclo[2.2.2.]octanes or 1,4-diazabicyclo[2.2.2]octanes from acyclic or heterocyclic amines or mixtures thereof, the improvement which comprises contacting said amines with high silica zeolite catalyst having silica to alumina ratio of at least about 20, preferably with a ZSM-5 type zeolite or with a zeolite having the following composition in th dehydrated state

$$0.01\text{-}2.0\ M_{\frac{2}{n}}O \quad : \quad Al_2O_3 \quad : \quad 20\text{-}100\ SiO_2$$

wherein M represents at least one cation of H, K Ca, Na, Mg, Zn or $UO_2$ and n represents the valence of M, said composition having a characteristic X-ray powder diffraction pattern having at least the interplanar spacings of Table B.